# EUROPEAN PATENT APPLICATION

(11) **EP 4 592 683 A1**
(43) Date of publication of application: **30.07.2025**
(21) Application number: 25151849.4
(22) Date of filing: 14.01.2025
(51) Int. Cl.: G01N 35/00

(54) **MONITORING METHOD, MONITORING PROGRAM, AND MONITORING APPARATUS**

(30) Priority: 26.01.2024 JP 2024010499
(71) Applicant: Yokogawa Electric Corporation, Tokyo 180-8750 (JP)
(72) Inventor: SAKASHITA, Hirofumi, Musashino-shi, Tokyo, 180-8750 (JP); MORI, Yuusuke, Musashino-shi, Tokyo, 180-8750 (JP); KOMETANI, Satoshi, Musashino-shi, Tokyo, 180-8750 (JP); MIYAMAE, Hirokazu, Musashino-shi, Tokyo, 180-8750 (JP); SHIMIZU, Yoshifumi, Musashino-shi, Tokyo, 180-8750 (JP)
(74) Representative: Stöckeler, Ferdinand

(57) **Abstract**

A monitoring method for monitoring an analysis apparatus (10) configured to observe a target object includes acquiring self-diagnostic data on the analysis apparatus (10) outside an observation period of the target object by the analysis apparatus (10), diagnosing the state of the analysis apparatus (10) based on the self-diagnostic data, and outputting a diagnostic result of the state of the analysis apparatus (10).

## Description

### TECHNICAL FIELD

The present disclosure relates to a monitoring method, a monitoring program, and a monitoring apparatus for an analysis apparatus.

### BACKGROUND

As described in Patent Literature (PTL) 1, a system that uploads information on an analysis apparatus and saves the information as shared data is known.

### CITATION LIST

### Patent Literature

PTL 1: JP 2003-344422 A

### SUMMARY

It is required not only to share information on an analysis apparatus, but also to use the information to maintain the state of the analysis apparatus.

It would be helpful to provide a monitoring method, a monitoring program, and a monitoring apparatus that can easily maintain the state of an analysis apparatus.
(1) A monitoring method according to some embodiments is a method for monitoring an analysis apparatus configured to observe a target object. The monitoring method includes:
   acquiring self-diagnostic data on the analysis apparatus in a self-diagnostic possible period during which the analysis apparatus is in a state of not executing observation of the target object;
   diagnosing a state of the analysis apparatus based on the self-diagnostic data; and
   outputting a diagnostic result of the state of the analysis apparatus.

Acquiring the self-diagnostic data during the period in which the analysis apparatus is in the state of not executing observation of the target object reduces an impact on an observation result of the target object. By outputting the diagnostic result of the state of the analysis apparatus, the state of the analysis apparatus can be more easily grasped than when the state of the analysis apparatus is grasped from the self-diagnostic data alone. As a result, the state of the analysis apparatus can be maintained easily.

(2) In the monitoring method as described in (1) above, the self-diagnostic possible period may include a period during which the analysis apparatus is not set to an observation mode.

(3) In the monitoring method as described in (1) or (2) above, when the analysis apparatus is operating in a continuous observation mode that is a mode of continuously executing observation of the target object, the self-diagnostic possible period may include a period before the analysis apparatus starts operations in the continuous observation mode, or a period after the analysis apparatus terminates the operations in the continuous observation mode.

(4) In the monitoring method as described in (1) or (2) above, when the analysis apparatus is operating in a time lapse mode that is a mode of intermittently executing observation of the target object, the self-diagnostic possible period may include at least part of a period before the analysis apparatus starts a series of operations to be executed in a section of a period during which the analysis apparatus is operating in the time lapse mode, or at least part of a period after the analysis apparatus terminates the series of operations.

(5) In the monitoring method as described in any one of (1) to (4) above, the self-diagnostic data may be acquired immediately after the self-diagnostic possible period is started, or immediately before the self-diagnostic possible period is terminated. This avoids a situation in which the acquisition of the self-diagnostic data is started when a user does not intend it. As a result, the user can perform operation safely.

(6) The monitoring method as described in any one of (1) to (5) above may further include accepting input from a user of the analysis apparatus to set time of acquiring the self-diagnostic data. The self-diagnostic data may be acquired at or after the time set by the user of the analysis apparatus, and acquired within the self-diagnostic possible period. By allowing the user to set the time of acquiring the self-diagnostic data, the user can check the state of the analysis apparatus without forgetting. As a result, the state of the analysis apparatus can be maintained easily.

(7) The monitoring method as described in any one of (1) to (6) above may further include acquiring, as the self-diagnostic data, data that can be measured by setting a jig on the analysis apparatus.

(8) In the monitoring method as described in (7) above, the jig may be provided with an aperture. When the analysis apparatus includes a camera, the aperture may be configured to enable analysis of noise contained in an image generated by the camera, or dust or dirt adhering to the camera, by imaging the aperture by the camera.

(9) In the monitoring method as described in (7) or (8) above, the jig may include a pattern area displaying a specific pattern. When the analysis apparatus includes a camera and a stage on which the target object can be placed, the pattern area may be configured to enable generation of an image to check the movement accuracy of the stage, by placing the jig on the stage, moving the stage to multiple positions, and imaging, by the camera, the specific pattern of the pattern area when the stage is moved to each of the positions.

(10) In the monitoring method as described in any one of (7) to (9) above, the jig may be provided with a holder. When the analysis apparatus has an autofocus function, the holder may be configured to enable placement of a sample used by the analysis apparatus to acquire an autofocus optical signal.

Acquire the data using the jig improves the diagnostic accuracy of the state of the analysis apparatus. As a result, the state of the analysis apparatus can be maintained easily.

(11) In the monitoring method as described in any one of (1) to (10) above, the self-diagnostic data may include data measured by a sensor provided in the analysis apparatus.

(12) In the monitoring method as described in (11) above, the sensor may include at least one of an illuminance sensor configured to measure the intensity of laser light used by the analysis apparatus to observe the target object, a temperature sensor configured to measure temperature inside a chamber in which the target object is placed, or a gas sensor configured to measure gas concentration inside the chamber.

When the data measured by the sensor provided in the analysis apparatus is acquired as the self-diagnostic data, the self-diagnostic data is acquired without using a jig. Acquiring the self-diagnostic data without using a jig allows the acquisition of the self-diagnostic data in a short time. As a result, the state of the analysis apparatus can be maintained easily.

(13) The monitoring method as described in any one of (1) to (12) above may further include notifying outside a network connected to the analysis apparatus of a result of analyzing the state of the analysis apparatus in the diagnostic result.

(14) The monitoring method as described in any one of (1) to (13) above may further include notifying outside a network connected to the analysis apparatus of the diagnostic result.

(15) The monitoring method as described in any one of (1) to (14) above may further include notifying a user of the analysis apparatus of the diagnostic result by displaying the diagnostic result.

(16) The monitoring method as described in any one of (1) to (15) above may further include notifying a user of the analysis apparatus of a result of analyzing the state of the analysis apparatus in the diagnostic result.

Notifying the state of the analysis apparatus allows the user of the analysis apparatus or a person in charge of maintenance of the analysis apparatus to check the diagnostic result, without operating any device connected to a network connected to the analysis apparatus. As a result, the state of the analysis apparatus can be maintained easily.

(17) The monitoring method as described in any one of (1) to (16) above may further include:
acquiring a log that records operations of the analysis apparatus in a period during which the analysis apparatus is observing the target object; and
outputting the log.

Since a control apparatus acquires the log from the analysis apparatus and outputs the log, the user of the analysis apparatus or the person in charge of maintenance of the analysis apparatus can check the operation state of the analysis apparatus without operating the control apparatus or the analysis apparatus.

(18) The monitoring method as described in (17) above may further include, when at least one item included in the log is out of a log monitoring criterion, notifying outside a network connected to the analysis apparatus of an analysis result of the log. This makes it possible for the user of the analysis apparatus or the person in charge of maintenance of the analysis apparatus to check an abnormality contained in the log of the analysis apparatus, without connecting to the network connected to the analysis apparatus. As a result, the state of the analysis apparatus can be maintained easily.

(19) A monitoring program according to some embodiments is configured to cause a processor to monitor an analysis apparatus configured to observe a target object. The monitoring program is configured to cause the processor to execute operations including:
acquiring self-diagnostic data on the analysis apparatus, outside an observation period of the target object by the analysis apparatus;
diagnosing a state of the analysis apparatus based on the self-diagnostic data; and
outputting a diagnostic result of the state of the analysis apparatus.

(20) A monitoring apparatus according to some embodiments is configured to monitor an analysis apparatus configured to observe a target object. The monitoring apparatus is configured to:
acquire self-diagnostic data on the analysis apparatus in a self-diagnostic possible period during which the analysis apparatus is in a state of not executing observation of the target object;
diagnose a state of the analysis apparatus based on the self-diagnostic data; and
output a diagnostic result of the state of the analysis apparatus.

According to the monitoring method, the monitoring program, and the monitoring apparatus of the present disclosure, the state of the analysis apparatus can be maintained easily.

### BRIEF DESCRIPTION OF THE DRAWINGS

In the accompanying drawings:
FIG. 1 is a block diagram illustrating an example of a configuration of a monitoring system according to a comparative example;
FIG. 2A is a block diagram illustrating an example of a configuration of a monitoring system according to an embodiment;
FIG. 2B is a block diagram illustrating an example of a configuration of a server controller;
FIG. 2C is a block diagram illustrating an example of a configuration of a sensor;
FIG. 3 is a schematic diagram illustrating an example of a configuration of a jig; and
FIG. 4 is a flowchart illustrating an example of a procedure in a monitoring method according to the embodiment.

### DETAILED DESCRIPTION

### (Comparative Example)

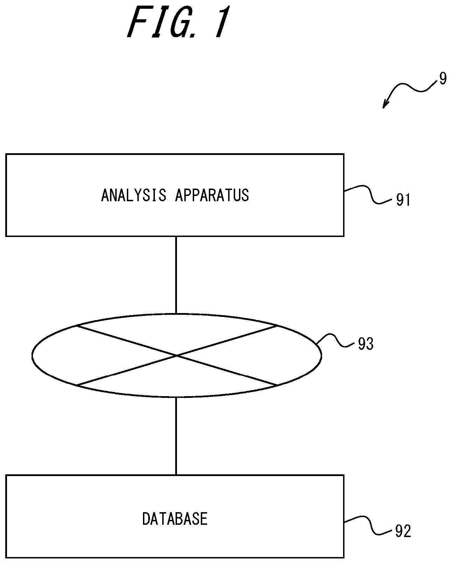

As illustrated in FIG. 1, a monitoring system 9 according to a comparative example includes an analysis apparatus 91 and a database 92. The analysis apparatus 91 and the database 92 are communicably connected via a network 93.

The analysis apparatus 91 is configured to be able to observe a target object with a microscope. The analysis apparatus 91 uploads, to the database 92, data representing the state of the analysis apparatus 91 itself during executing operations of observing the target object. The database 92 accumulates the data representing the state of the analysis apparatus 91. A user of the analysis apparatus 91 manages, based on the data accumulated in the database 92, consumable supplies for the analysis apparatus 91 and a maintenance schedule for the analysis apparatus 91.

However, the information accumulated in the database 92 is only shared, and is not specifically analyzed or examined.

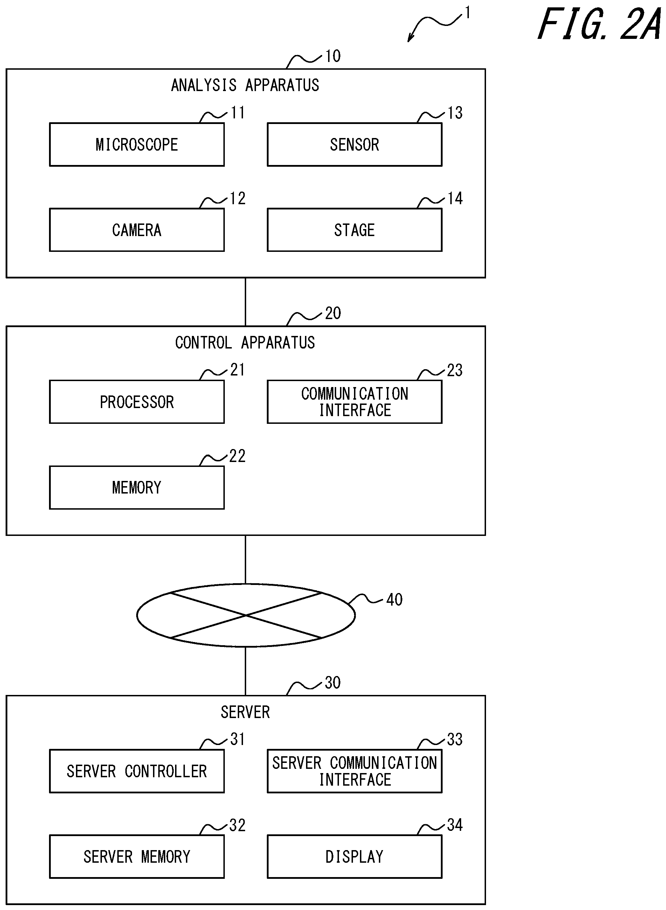

A monitoring system 1 (refer to FIG. 2A) according to the present disclosure utilizes data representing the state of an analysis apparatus 10 (refer to FIG. 2A) to achieve easy maintenance of the state of the analysis apparatus 10. The monitoring system 1 and a monitoring method according to the present disclosure will be described below.

### (Example of Configuration of Monitoring System 1 According to Present Disclosure)

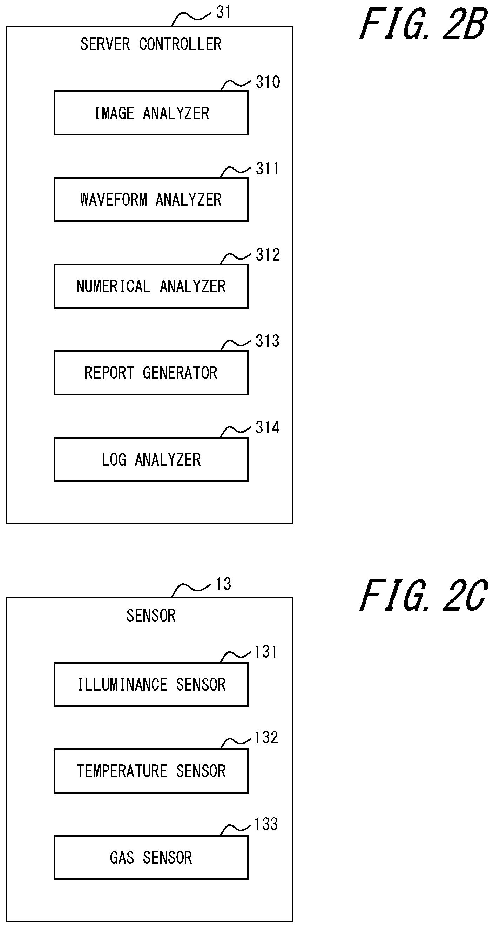

As illustrated in FIGS. 2A, 2B, and 2C, the monitoring system 1 according to an embodiment of the present disclosure includes the analysis apparatus 10, a control apparatus 20, and a server 30. The control apparatus 20 controls the analysis apparatus 10. The control apparatus 20 may be configured integrally with the analysis apparatus 10. In other words, the control apparatus 20 may be embedded in the analysis apparatus 10. At least part of the control apparatus 20 or at least part of the server 30 is also referred to as a monitoring apparatus. In other words, the monitoring apparatus may be configured with at least part of the control apparatus 20 or at least part of the server 30.

The control apparatus 20 and the server 30 are connected via a network 40 communicably by wired or wireless communication. When the control apparatus 20 is embedded in the analysis apparatus 10, the analysis apparatus 10 and the server 30 are connected via the network 40 communicably by wired or wireless communication. The network 40 may be the Internet. The network 40 may be a closed network using dedicated lines that are separated from the Internet. When the network 40 is the Internet, a virtual private network (VPN) may be used to ensure the security of communication between the control apparatus 20 and the server 30.

In the monitoring system 1, the analysis apparatus 10 acquires data regarding a target object by analyzing images of the target object. The analysis apparatus 10 may be, for example, a high content analysis (HCA) device. The target object may be, for example, living cells. The analysis apparatus 10 may be configured to be able to observe chronological changes in the living cells, or to enable observation while the living cells are being cultured. The target object analyzed by the analysis apparatus 10 is not limited to the living cells, but may be various other objects.

The control apparatus 20 acquires the data regarding the target object by controlling the analysis apparatus 10, and also acquires data representing the state of the analysis apparatus 10. The data representing the state of the analysis apparatus 10 is data used for diagnosing the analysis apparatus 10, and is also referred to as self-diagnostic data. The control apparatus 20 transmits the self-diagnostic data to the server 30.

The server 30 diagnoses the state of the analysis apparatus 10 based on the self-diagnostic data, and outputs a diagnostic result. With reference to the diagnostic result, a user, such as an administrator or an operator, of the analysis apparatus 10 can check the reproducibility of the data regarding the target object, which is obtained by the analysis apparatus 10. When the target object is living cells, the user of the analysis apparatus 10 can check, with reference to the diagnostic result, the stability of environment for culturing the living cells. In other words, the user of the analysis apparatus 10 can check the quality of the data regarding the target object, which is obtained by the analysis apparatus 10, based on the diagnostic result.

The server 30 may automatically perform maintenance of the analysis apparatus 10 based on the diagnostic result. For example, the server 30 may perform maintenance of the analysis apparatus 10 by instructing the control apparatus 20 to replace parts or consumable supplies of the analysis apparatus 10.

The analysis apparatus 10 may be monitored by a company that is contracted to perform maintenance. In this case, a person in charge at the company contracted to perform maintenance may check, on the server 30, diagnostic results for multiple analysis apparatuses 10 that are subjected to maintenance, may determine whether the maintenance of each analysis apparatus 10 is necessary based on the diagnostic results, and may perform maintenance of each analysis apparatus 10.

By automating or outsourcing the maintenance of the analysis apparatus 10, the user of the analysis apparatus 10 can reliably use the analysis apparatus 10 in a state of assuring the quality of the data regarding the target object. In addition, when the analysis apparatus 10 is managed with reference to the diagnostic result obtained based on the self-diagnostic data, the state of the analysis apparatus 10 is more easily maintained than when the analysis apparatus 10 is managed with reference to the self-diagnostic data on the analysis apparatus 10 alone.

A specific example of each component of the monitoring system 1 will be described below.

### <Analysis Apparatus 10>

As illustrated in FIG. 2A, the analysis apparatus 10 includes a microscope 11, a stage 14, a camera 12, and a sensor 13.

The microscope 11 has an optical system that forms an image of the target object on an imaging surface of the camera 12. The optical system includes an objective lens located on the side of the target object. The microscope 11 may be configured to be able to change magnification when forming the image of the target object. The microscope 11 may be configured to be able to adjust the focus of the optical system when forming the image of the target object. The microscope 11 may be configured with an autofocus function that allows the focus of the optical system to be adjusted automatically.

In the present disclosure, the microscope 11 is a fluorescence microscope using laser light. When the microscope 11 is the fluorescence microscope using the laser light, the microscope 11 has a light source that emits the laser light. The microscope 11 may be configured in various other aspects, not limited to the fluorescence microscope using the laser light.

The camera 12 captures the image of the target object, which is formed by the microscope 11, and outputs the image. The camera 12 may be configured with an image sensor, such as a charge coupled device (CCD) image sensor or complementary metal oxide semiconductor (CMOS) sensor. The imaging surface on which the microscope 11 forms the image of the target object may be a light-receiving surface of the image sensor.

The camera 12 may be cooled to reduce noise contained in the captured image. The camera 12 may be configured with a cooling means, such as air cooler or water cooler. The camera 12 may be configured to be able to measure the temperature of the camera 12 itself. The camera 12 may be configured to be able to control the temperature of the camera 12 itself.

The camera 12 may be configured with an optical system that forms an image of the target object. The camera 12 may be configured to allow the focus of the optical system to be adjusted. The camera 12 may have an autofocus function that allows the focus of the optical system to be adjusted automatically.

The stage 14 is configured to allow placement of the target object. The stage 14 may be included in the microscope 11. The stage 14 may be configured to move relative to the microscope 11 and the camera 12. Conversely, the microscope 11 and the camera 12 may be configured to move relative to the stage 14. Both the stage 14 and the microscope 11 and camera 12 may be configured to be movable.

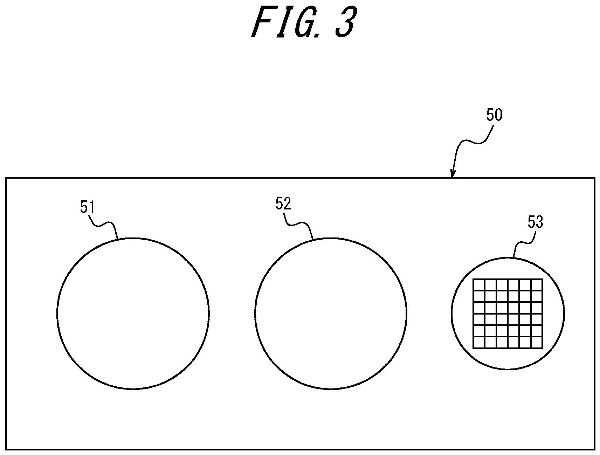

The stage 14 may be configured to allow placement of a container in which the target object is contained. The stage 14 may be configured to allow placement of a jig 50 (refer to FIG. 3) used to acquire the self-diagnostic data.

As illustrated in FIG. 3, the jig 50 includes a holder 51, an aperture 52, and a pattern area 53 that displays a specific pattern. The jig 50 may not include at least one of the holder 51, the aperture 52, or the pattern area 53.

The holder 51 is configured to allow placement of a 35 mm dish or the like, which is used to contain cells as the target object. When placing the 35 mm dish on the holder 51, the 35 mm dish may be filled with only water. The stage 14 is moved to the position of the holder 51 so that the 35 mm dish can be observed with the objective lens of the microscope 11, and autofocus is performed on the 35 mm dish filled with only water, to acquire an autofocus optical signal. The holder 51 is configured to allow placement of a sample for the analysis apparatus 10 to acquire the autofocus optical signal.

The aperture 52 is configured so that the camera 12 can image a space in which nothing exists. An image of the aperture 52 captured by the camera 12 includes noise from the image sensor of the camera 12, dust or dirt adhering to the microscope 11 or the camera 12, or the like. In other words, by moving the stage 14 to the position of the aperture 52 and capturing an image, information on the noise, or the dust or dirt superimposed on the captured image is acquired. The aperture 52 is configured so that, by capturing the image of the aperture 52 by the camera 12, the noise contained in images generated by the camera 12, or the dust or dirt adhering to the camera 12 can be analyzed.

The pattern area 53 displays a specific pattern. The specific pattern may include, for example, a grid pattern. The pattern area 53 is wider than the field of view of the obj ective lens of the microscope 11. By imaging the pattern area 53 in multiple fields of view while moving the stage 14, an image to check the movement accuracy of the stage 14 is acquired. The movement accuracy of the stage 14 is checked by the connection of the specific pattern in images of the respective fields of view. The pattern area 53 is configured so that the image to check the movement accuracy of the stage 14 can be generated by placing the jig 50 on the stage 14, moving the stage 14 to multiple positions, and capturing, by the camera 12, an image of the pattern area 53 when the stage 14 is moved to each position.

When the target object is cells, the stage 14 may include a chamber that can control the temperature, carbon dioxide concentration, or the like necessary for culturing the cells or maintaining the state of the cells.

The sensor 13 measures a physical quantity that indicates the state of each part of the analysis apparatus 10, and outputs measurement results as the self-diagnostic data. As illustrated in FIG. 2C, the sensor 13 includes an illuminance sensor 131, a temperature sensor 132, and a gas sensor 133.

When the microscope 11 is a fluorescence microscope using laser light, the illuminance sensor 131 is configured to measure the intensity of the laser light emitted from a light source. The illuminance sensor 131 may be located on the stage 14. When the illuminance sensor 131 is located on the stage 14, the stage 14 may be controlled so that the illuminance sensor 131 is moved to an irradiation position of the laser light.

The temperature sensor 132 is configured to measure the temperature of the stage 14 or the chamber on or in which the target object is placed.

The gas sensor 133 is configured to measure the gas concentration, such as the carbon dioxide concentration, of the stage 14 on which the target object is placed.

The sensor 13 may include at least one of the illuminance sensor 131, the temperature sensor 132, or the gas sensor 133. Conversely, the sensor 13 may not include at least one of the illuminance sensor 131, the temperature sensor 132, or the gas sensor 133. The sensor 13 may be configured to be able to measure various other physical quantities, not limited to these examples.

### <Control Apparatus 20>

The control apparatus 20 includes a processor 21, a memory 22, and a communication interface 23.

The processor 21 may be configured with, for example, a central processing unit (CPU) or the like. The processor 21 may realize various functions of the control apparatus 20 by executing a predetermined program. Specifically, the processor 21 may acquire an image of any part of the target object by controlling the microscope 11 of the analysis apparatus 10, or the stage 14 and the camera 12 of the analysis apparatus 10. The processor 21 may measure the intensity of the laser light with the illuminance sensor 131 by controlling the stage 14.

The memory 22 may store various types of information used in operations of the control apparatus 20, programs to realize the functions of the control apparatus 20, or the like. The memory 22 may function as work memory for the processor 21. The memory 22 may be configured with an electromagnetic storage medium such as a magnetic disk, or a memory such as a semiconductor memory or a magnetic memory. The memory 22 may be configured integrally with the processor 21. The memory 22 may be configured as a memory device separate from the control apparatus 20.

The communication interface 23 is configured with a communication device that is connected to the analysis apparatus 10 and the server 30 communicably by wired or wireless communication. The communication device may be configured communicably based on, for example, a communication standard for a local area network (LAN). The communication device may be configured communicably based on, for example, a mobile communication standard, such as the 4th generation (4G) standard or long term evolution (LTE) standard or the 5th generation (5G) standard. The communication device may be configured communicably based on various communication standards, not limited to these examples.

The control apparatus 20 may be configured as a personal computer (PC). The control apparatus 20 may be configured in various other aspects.

The control apparatus 20 may include a display device to notify the user of the analysis apparatus 10 of information. The display device may include, for example, a liquid crystal display (LCD). The display device may include, for example, an organic electro-luminescence (EL) display or an inorganic EL display. The display device may include various other types of displays, not limited to these displays. The display device may also include light emitting devices such as light emitting diodes (LEDs).

The control apparatus 20 may include an audio output device, such as a speaker, to notify the user of the analysis apparatus 10 of information. The server 30 may include other output devices.

The control apparatus 20 may include an input device to receive input of operation from the user of the analysis apparatus 10, or input of data or the like. The input device may include, for example, a keyboard or physical keys, or may include a touch panel or a touch sensor, or a pointing device such as a mouse. The input device may include various other devices, not limited to these examples.

### <Server 30>

The server 30 includes a server controller 31, a server memory 32, a server communication interface 33, and a display 34.

The server controller 31 may be configured with a processor such as a central processing unit (CPU). The server controller 31 may realize various functions of the server 30 by executing a predetermined program. As illustrated in FIG. 2B, the server controller 31 includes an image analyzer 310, a waveform analyzer 311, a numerical analyzer 312, a report generator 313, and a log analyzer 314. Operations of each part are described later. The server controller 31 may not include at least one of the image analyzer 310, the waveform analyzer 311, the numerical analyzer 312, the report generator 313, or the log analyzer 314.

The server memory 32 may store various types of information used in operations of the server 30, programs to realize the functions of the server 30, or the like. The server memory 32 may function as work memory for the server controller 31. The server memory 32 may be configured with an electromagnetic storage medium such as a magnetic disk, or a memory such as a semiconductor memory or a magnetic memory. The server memory 32 may be configured integrally with the server controller 31. The server memory 32 may be configured as a memory device separate from the server 30.

The server communication interface 33 is configured with a communication device that is connected to the control apparatus 20 or the analysis apparatus 10 communicably by wired or wireless communication. The communication device may be configured communicably based on, for example, a communication standard for a LAN. The communication device may be configured communicably based on, for example, a mobile communication standard, such as the 4G standard or LTE standard or the 5G standard. The communication device may be configured communicably based on various communication standards, not limited to these examples.

The display 34 is configured to notify information to the user of the analysis apparatus 10 or the person in charge of maintenance of the analysis apparatus 10. The display 34 may include, for example, an LCD, an organic EL display, or an inorganic EL display. The display 34 may include various other types of displays, not limited to these displays.

The server 30 may include an input device to receive input of data or the like from the user of the analysis apparatus 10 or the person in charge of maintenance of the analysis apparatus 10. The input device may include, for example, a keyboard or physical keys, or may include a touch panel or a touch sensor, or a pointing device such as a mouse. The input device may include various other devices, not limited to these examples.

The server 30 may be configured as a PC, or at least one server apparatus. The server 30 may be realized as a cloud computing system.

### (Example of Operations of Monitoring State of Analysis Apparatus 10)

The monitoring system 1 monitors the state of the analysis apparatus 10. In the monitoring system 1, the control apparatus 20 acquires, using the sensor 13 and the like of the analysis apparatus 10, the self-diagnostic data on the analysis apparatus 10 in a self-diagnostic possible period. The self-diagnostic possible period is a period during which the control apparatus 20 can acquire the self-diagnostic data on the analysis apparatus 10. In the present disclosure, the self-diagnostic possible period is a period during which the analysis apparatus 10 is in a state of not executing observation of the target object. Acquiring the self-diagnostic data, while the analysis apparatus is not executing observation of the target object, reduces an impact of operations of acquiring the self-diagnostic data on data regarding the target object, which is obtained by the analysis apparatus 10 executing observation of the target object.

When executing observation of the target object, the analysis apparatus 10 may operate in either a continuous observation mode or a time lapse mode.

The continuous observation mode is a mode in which the analysis apparatus 10 continuously executes observation of the target object. The continuous observation may include, for example, imaging the target object at a predetermined frame rate, such as 60 frames per second. The continuous observation may include continuous imaging by operating a motor of the microscope.

The time lapse mode is a mode in which the analysis apparatus 10 intermittently executes observation of the target object. The intermittent observation corresponds to imaging the target object at time intervals longer than time intervals for imaging the target object in the continuous observation mode. The time intervals for imaging the target object in the time lapse mode may be set in units of one minute or one hour, for example, but may be set as appropriate, not limited to this.

A state in which the analysis apparatus 10 is operating in either the continuous observation mode or the time lapse mode corresponds to a state in which the analysis apparatus 10 is executing operations of observing the target object. Conversely, a state in which the analysis apparatus 10 is not executing the operations of observing the target object corresponds to a state in which the analysis apparatus 10 is not operating in either the continuous observation mode or the time lapse mode.

In the present disclosure, the continuous observation mode and the time lapse mode are collectively referred to as an observation mode. The analysis apparatus 10 may execute observation of the target object by operating in a set observation mode. Conversely, a period in which the analysis apparatus 10 is not set in the observation mode is a period in which the analysis apparatus 10 is not executing the operations of observing the target object. The self-diagnostic possible period may include the period in which the analysis apparatus 10 is not set in the observation mode.

When the analysis apparatus 10 is operating in the continuous observation mode, the self-diagnostic possible period may include at least part of a period before the analysis apparatus 10 starts operations in the continuous observation mode, or at least part of a period after the analysis apparatus 10 terminates the operations in the continuous observation mode.

When the analysis apparatus 10 is operating in the time lapse mode, the self-diagnostic possible period may include at least part of a period before the analysis apparatus 10 starts a series of operations to be executed in a section of a period during which the analysis apparatus 10 is operating in the time lapse mode, or at least part of a period after the analysis apparatus 10 terminates the series of operations to be executed in the section of the period during which the analysis apparatus 10 is operating in the time lapse mode. The section of the period during which the analysis apparatus 10 is operating in the time lapse mode is a period during which the analysis apparatus 10 executes a single time of observation that has been started intermittently at the set time interval. The termination of the series of operations in the section of operations in the time lapse mode means that the analysis apparatus 10 enters a standby state until a start of operations in the next section. In other words, when the analysis apparatus 10 is operating in the time lapse mode, the self-diagnostic possible period may include a period during which the analysis apparatus 10 is temporarily paused while intermittently executing observation of the target object. The period during which the analysis apparatus 10 is temporarily paused while intermittently executing observation of the target object is also referred to as a pause period.

In addition, when the analysis apparatus 10 is operating in the time lapse mode, the self-diagnostic possible period may include at least part of a period before the analysis apparatus 10 starts the operations in the time lapse mode, or at least part of a period after the analysis apparatus 10 terminates the operations in the time lapse mode. In other words, when the analysis apparatus 10 is operating in the time lapse mode, the self-diagnostic possible period may not include the pause period.

Whether the self-diagnostic possible period includes the pause period may be determined so that the self-diagnostic data is acquired at an appropriate frequency, according to the time intervals at which the analysis apparatus 10 images the target object in the time lapse mode.

The control apparatus 20 uploads, to the server 30, the self-diagnostic data on the analysis apparatus 10, which has been acquired in the self-diagnostic possible period. The server 30 diagnoses the state of the analysis apparatus 10 based on the self-diagnostic data. The server 30 outputs a diagnostic result so that the user or the person in charge of maintenance can check the diagnostic result.

In the monitoring system 1 according to the present embodiment, an example of operations of the control apparatus 20 and the server 30 monitoring the state of the analysis apparatus 10 will be described below.

### <Example of Operations of Control Apparatus 20>

The control apparatus 20 controls the analysis apparatus 10 and also monitors the state of the analysis apparatus 10.

Operations of the control apparatus 20 controlling the analysis apparatus 10 are achieved by the processor 21 of the control apparatus 20 executing control software. As operations of the control software, the processor 21 acquires the self-diagnostic data on the analysis apparatus 10 via the communication interface 23, and stores the self-diagnostic data in the memory 22.

Operations of the control apparatus 20 monitoring the analysis apparatus 10 are achieved by the processor 21 executing monitoring software. When executing the monitoring software, the control apparatus 20 constitutes at least part of the monitoring apparatus. As operations of the monitoring software, the processor 21 detects that the self-diagnostic data on the analysis apparatus 10 has been stored in the memory 22, and uploads the self-diagnostic data on the analysis apparatus 10 to the server 30 via the network 40. The network 40 used to upload the self-diagnostic data may be a closed network.

The processor 21 may be configured to execute both the control software and the monitoring software using a single CPU or the like. The processor 21 may be configured to execute the control software and the monitoring software using separate CPUs or the like. The control software and the monitoring software may be implemented as a single software program.

The server 30 receives, by the server communication interface 33, the self-diagnostic data on the analysis apparatus 10 uploaded from the control apparatus 20. The server controller 31 analyzes the self-diagnostic data on the analysis apparatus 10 using the image analyzer 310, the waveform analyzer 311, or the numerical analyzer 312 (refer to FIG. 2B). The server controller 31 may store the self-diagnostic data on the analysis apparatus 10 in the server memory 32, and analyze the self-diagnostic data.

The server controller 31 generates, by the report generator 313 (refer to FIG. 2B), a diagnostic result of the analysis apparatus 10 based on the self-diagnostic data. The server controller 31 may generate the diagnostic result as a file in a predetermined format, such as PDF. The server controller 31 may generate the diagnostic result as web contents in a predetermined format, such as hypertext markup language (HTML).

The server controller 31 may display the diagnostic result on the display 34. The server controller 31 may store the diagnostic result in the server memory 32, and display, on the display 34, information such as a uniform resource locator (URL) that specifies a location at which the diagnostic result is stored, so that the user can view the diagnostic result on a terminal apparatus. This allows the user of the analysis apparatus 10 to check the diagnostic result, without going to a location in which the control apparatus 20 or the analysis apparatus 10 is installed.

When the maintenance of the analysis apparatus 10 is outsourced, the person in charge at the company that is contracted to perform the maintenance can check the diagnostic result, without going to the location in which the control apparatus 20 or the analysis apparatus 10 is installed. When the person is in charge of maintenance of multiple analysis apparatuses 10, the person in charge of maintenance may check diagnostic results for the multiple analysis apparatuses 10 together on the display 34. The person in charge of maintenance can check the diagnostic results, without going to installation locations of the respective analysis apparatuses 10, and only needs to go to an installation location of an analysis apparatus 10 that requires maintenance. As a result, the states of the analysis apparatuses 10 can be maintained easily.

The server controller 31 may transmit the diagnostic result from the server communication interface 33 to the control apparatus 20 via the network 40. The network 40, which is used to transmit the diagnostic result to the control apparatus 20, may be a closed network. The processor 21 of the control apparatus 20 may acquire the diagnostic result, and output the diagnostic result on the control apparatus 20 or the analysis apparatus 10. When the control apparatus 20 or the analysis apparatus 10 includes a display device, the diagnostic result may be displayed thereon. The user of the analysis apparatus 10 can check the diagnostic result on the control apparatus 20 or the analysis apparatus 10. In other words, there is no need to prepare an external device to be used to check the diagnostic result. As a result, the state of the analysis apparatus 10 can be maintained easily.

The server controller 31 may transmit the diagnostic result from the server communication interface 33 to an external apparatus, such as a terminal apparatus of the user of the analysis apparatus 10 or the person in charge of maintenance of the analysis apparatus 10. The user of the analysis apparatus 10 or the person in charge of maintenance of the analysis apparatus 10 can check the diagnostic result, without going to a location in which the analysis apparatus 10 is installed. As a result, the state of the analysis apparatus 10 can be maintained easily.

When it is determined based on the diagnostic result that the maintenance of the analysis apparatus 10 is required, the server controller 31 may notify the external apparatus, such as the terminal apparatus of the user of the analysis apparatus 10 or the person in charge of maintenance of the analysis apparatus 10, that the analysis apparatus 10 requires maintenance. In other words, the server controller 31 may notify outside the network 40 connected to the analysis apparatus 10 that the analysis apparatus 10 requires maintenance. This allows the user of the analysis apparatus 10 or the person in charge of maintenance of the analysis apparatus 10 can check that the analysis apparatus 10 requires maintenance, without connecting to the network 40 connected to the analysis apparatus 10. As a result, the state of the analysis apparatus 10 can be maintained easily.

The server controller 31 may determine that the maintenance of the analysis apparatus 10 is required when the diagnostic result includes an error. The error in the diagnostic result may include that at least one item indicating the state of the analysis apparatus 10 is abnormal. In other words, the error in the diagnostic result may include that at least one item indicating the state of the analysis apparatus 10 is out of a monitoring criterion. When a measurement result of an item indicating the state of the analysis apparatus 10 is obtained as a numerical value, the monitoring criterion for the item may be set as a numerical range. When a measurement result of an item indicating the state of the analysis apparatus 10 is obtained as an image or a waveform, the monitoring criterion for the item may be set as a reference image or a reference waveform. The reference image or the reference waveform is an image or a waveform that is used as a criterion for monitoring the state of the analysis apparatus 10. The server controller 31 may generate a model for machine learning or deep learning using the reference image or the reference waveform, and determine, using the generated model, whether at least one item indicating the state of the analysis apparatus 10 is out of the monitoring criterion.

The server controller 31 may notify the user of the analysis apparatus 10 or the person in charge of maintenance of the analysis apparatus 10, by sending an e-mail or message, that the maintenance of the analysis apparatus 10 is required. When notifying that the maintenance of the analysis apparatus 10 is required, the server controller 31 may attach a file such as a PDF file generated as the diagnostic result. By notifying that the maintenance of the analysis apparatus 10 is required, the user of the analysis apparatus 10 or the person in charge of maintenance of the analysis apparatus 10 does not have to check the entire diagnostic result. As a result, the state of the analysis apparatus 10 is maintained easily.

The server controller 31 may analyze the state of the analysis apparatus 10 in the diagnostic result, and notify outside the network 40 connected to the analysis apparatus 10 of an analysis result of the state of the analysis apparatus 10. The server controller 31 may notify outside the network 40 connected to the analysis apparatus 10 of the diagnostic result, regardless of whether at least one item indicating the state of the analysis apparatus 10 in the diagnostic result is out of a monitoring criterion. When at least one item indicating the state of the analysis apparatus 10 in the diagnostic result is out of the monitoring criterion, the server controller 31 may notify outside the network 40 connected to the analysis apparatus 10 of the diagnostic result. This allows the user of the analysis apparatus 10 or the person in charge of maintenance of the analysis apparatus 10 can check the diagnostic result, without operating a device connected to the network 40 connected to the analysis apparatus 10. As a result, the state of the analysis apparatus 10 can be maintained easily.

As described above, the server controller 31 monitors the state of the analysis apparatus 10 by diagnosing the state of the analysis apparatus 10 based on the self-diagnostic data on the analysis apparatus 10. The server 30 constitutes at least part of the monitoring apparatus.

### <Example of Self-Diagnostic Data>

As described above, the state of the analysis apparatus 10 is maintained easily by outputting the diagnostic result based on the self-diagnostic data on the analysis apparatus 10. An example of the self-diagnostic data used to diagnose the state of the analysis apparatus 10 will be described below.

### «Self-Diagnostic Data Acquired Without Using Jig 50»

The self-diagnostic data on the analysis apparatus 10 includes data acquired without using the jig 50. Examples of the self-diagnostic data acquired without using the jig 50 will be described below.

When the analysis apparatus 10 observes the target object using laser light, the self-diagnostic data on the analysis apparatus 10 may include a result of measuring the intensity of the laser light with the illuminance sensor 131. When executing observation of the target object, the control apparatus 20 controls the analysis apparatus 10 to irradiate the target object with the laser light, but when measuring the intensity of the laser light, the control apparatus 20 controls the analysis apparatus 10 to irradiate the illuminance sensor 131 with the laser light. Specifically, the control apparatus 20 may move a light source of the laser light to the position of the illuminance sensor 131. The control apparatus 20 may move the illuminance sensor 131 to the irradiation position of the laser light.

When the analysis apparatus 10 controls the temperature of a chamber in order to culture living cells or maintain the state of living cells, the self-diagnostic data on the analysis apparatus 10 may include a result of measuring the temperature of the chamber with the temperature sensor 132. The temperature sensor 132 may be installed in the chamber.

When the analysis apparatus 10 controls the carbon dioxide concentration in the chamber, the self-diagnostic data on the analysis apparatus 10 may include a result of measuring the carbon dioxide concentration in the chamber with the gas sensor 133. The gas sensor 133 may be installed in the chamber.

The self-diagnostic data on the analysis apparatus 10 may include a measurement result of the temperature of the camera 12. The control apparatus 20 may acquire the measurement result of the temperature of the camera 12 from the camera 12 via an application programming interface (API) of the camera 12.

The server 30 analyzes, by the numerical analyzer 312 of the server controller 31, the measurement result of the intensity of the laser light, the temperature or carbon dioxide concentration of the chamber, the temperature of the camera 12, or the like, and generates a diagnostic result of the analysis apparatus 10. The numerical analyzer 312 may generate, as the diagnostic result of the analysis apparatus 10, as to whether a numerical value obtained as the measurement result for each item is within a predetermined range.

When data measured by the sensor 13 provided in the analysis apparatus 10 is acquired as the self-diagnostic data, the self-diagnostic data is acquired without using the jig 50. By acquiring the self-diagnostic data without using the jig 50, the self-diagnostic data is acquired in a short time. As a result, the state of the analysis apparatus 10 is maintained easily.

### «Self-Diagnostic Data Acquired Using Jig 50»

The self-diagnostic data on the analysis apparatus 10 may include data acquired using the jig 50. Examples of the self-diagnostic data acquired using the jig 50 will be described below.

The self-diagnostic data on the analysis apparatus 10 may include information on noise superimposed on images of the target object captured by the camera 12, or dust or dirt captured in the images. The information on the noise, dust, or dirt is acquired by imaging the aperture 52 of the jig 50 with the camera 12. The control apparatus 20 may prompt the user to place the jig 50 on the analysis apparatus 10 or control the analysis apparatus 10 to automatically place the jig 50, so that the analysis apparatus 10 can image the aperture 52 with the camera 12.

The self-diagnostic data on the analysis apparatus 10 may include an image to check the movement accuracy of the stage 14. The movement accuracy of the stage 14 affects the accuracy of images of the target object when the target object that does not fit in the field of view of the objective lens of the microscope 11 is divided into multiple fields of view and imaged with the camera 12, and an image of the target object is generated by connecting the images of the multiple fields of view. The image to check the movement accuracy of stage 14 is acquired by imaging the pattern area 53 divided into multiple fields of view, while moving the stage 14. The control apparatus 20 may prompt the user to place the jig 50 on the analysis apparatus 10 or control the analysis apparatus 10 to automatically place the jig 50, so that the pattern area 53 can be imaged with the camera 12.

The self-diagnostic data on the analysis apparatus 10 may include an autofocus optical signal. The autofocus optical signal is acquired by performing autofocus on a 35 mm dish filled with only water. The 35 mm dish is placed in the holder 51 of the jig 50. The control apparatus 20 may prompt the user to place the jig 50 on the analysis apparatus 10 or control the analysis apparatus 10 to automatically place the jig 50, so that autofocus can be performed on the 35 mm dish.

The server 30 analyzes, by the image analyzer 310 of the server controller 31, the image of the aperture 52, the images of the pattern area 53, or the like, and generates a diagnostic result of the analysis apparatus 10. The image analyzer 310 may generate, as the diagnostic result of the analysis apparatus 10, whether the image of the aperture 52 contains noise, dust, or dirt. The image analyzer 310 may generate, as the diagnostic result of the analysis apparatus 10, whether misalignment of a grid pattern in the images of the pattern area 53 is within a predetermined range. The server 30 analyzes, by the waveform analyzer 311 of the server controller 31, a waveform or the like of the autofocus optical signal, and generates a diagnostic result of the analysis apparatus 10. The waveform analyzer 311 may generate, as the diagnostic result of the analysis apparatus 10, whether the waveform of the autofocus optical signal is normal.

The data that can be acquired using the jig 50 is data that cannot be acquired by the analysis apparatus 10 in a state of executing normal observation operations. Acquiring the data using the jig 50 can improve the diagnostic accuracy of the state of the analysis apparatus 10. As a result, the state of the analysis apparatus 10 can be maintained easily.

### <Time to Acquire Self-Diagnostic Data>

As described above, the control apparatus 20 acquires the self-diagnostic data on the analysis apparatus 10 in the self-diagnostic possible period of the analysis apparatus 10. Time to acquire the self-diagnostic data may be set as appropriate in the self-diagnostic possible period of the analysis apparatus 10.

The control apparatus 20 may accept input from the user to set time to acquire the self-diagnostic data. In a case in which the time set by the user is within the self-diagnostic possible period, the control apparatus 20 may acquire the self-diagnostic data at the time set by the user. In a case in which the time set by the user is out of the self-diagnostic possible period, the control apparatus 20 may acquire the self-diagnostic data in the self-diagnostic possible period at or after the time set by the user. In other words, the self-diagnostic data may be acquired at or after the time set by the user and acquired within the self-diagnostic possible period. The control apparatus 20 may acquire both the data that can be acquired without using the jig 50 and the data that can be acquired using the jig 50. The control apparatus 20 may acquire only the data that can be acquired without using the jig 50, at the time set by the user. By allowing the user to set the time to acquire the self-diagnostic data, the user can check the state of the analysis apparatus 10 without forgetting. As a result, the state of the analysis apparatus 10 can be maintained easily.

The control apparatus 20 may acquire the self-diagnostic data, as interrupt operations, immediately before the analysis apparatus 10 starts the operations of observing the target object or immediately after the analysis apparatus 10 terminates the operations of observing the target object. In other words, the self-diagnostic data may be acquired at the time of starting or terminating the period during which the analysis apparatus 10 is executing observation of the target object. By acquiring the self-diagnostic data at the time of starting or terminating the observation period, the quality of an observation result acquired during the observation period is assured. The self-diagnostic data may be acquired immediately after a start of the self-diagnostic possible period or immediately before a termination of the self-diagnostic possible period. Upon recognizing that the analysis apparatus 10 is about to start the operations of observing the target object, the control apparatus 20 may acquire the self-diagnostic data before the analysis apparatus 10 starts the operations of observing the target object. The control apparatus 20 may also control the analysis apparatus 10 to start the operations of observing the target object, after the acquisition of the self-diagnostic data. The control apparatus 20 can thereby acquire the self-diagnostic data immediately before the analysis apparatus 10 starts the operations of observing the target object.

Acquiring the self-diagnostic data at the time of starting or terminating the period during which the analysis apparatus 10 is executing observation of the target object can correspond to executing a self-diagnosis together with the original purpose of the user executing the observation. Executing the self-diagnosis when the user executes an operation to start or terminate observation can avoid a situation in which the acquisition of the self-diagnostic data is started when the user does not intend it. For example, it is possible to avoid a situation in which the analysis apparatus 10 starts moving suddenly, without the user's intention, when the person in charge of maintenance is executing maintenance on the analysis apparatus 10. By not acquiring the self-diagnostic data when the user does not intend it, the user can perform operation safely.

### <Example of Procedure of Monitoring Method>

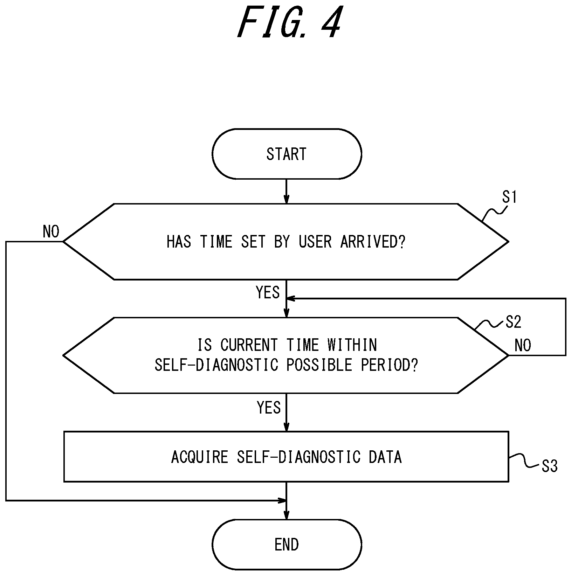

The processor 21 of the control apparatus 20 may execute a monitoring method that includes a procedure illustrated in the flowchart of FIG. 4. The procedure illustrated in the flowchart of FIG. 4 may be achieved as a monitoring program that is executed by a processor that constitutes the processor 21. The monitoring program may be stored in a non-temporary computer readable medium, such as an electromagnetic storage medium.

The processor 21 determines whether time set by the user, as time to acquire self-diagnostic data, has arrived (step S1). When the time set by the user has not arrived (step S 1: NO), the processor 21 terminates execution of the procedure in the flowchart in FIG. 4.

When the time set by the user has arrived (step S 1: YES), the processor 21 determines whether the current time is within a self-diagnostic possible period (step S2). Specifically, the processor 21 determines that the current time is out of the self-diagnostic possible period when the analysis apparatus 10 is operating in either of the continuous observation mode or the time lapse mode. Conversely, the processor 21 determines that the current time is within the self-diagnostic possible period when the analysis apparatus 10 is not operating in either of the continuous observation mode or the time lapse mode.

When the current time is not within the self-diagnostic possible period (step S2: NO), the processor 21 repeats the determination process of step S2 until the analysis apparatus 10 comes into a state of not executing observation operations, that is, until the analysis apparatus 10 terminates the observation operations. When the current time is within the self-diagnostic possible period (step S2: YES), the processor 21 acquires self-diagnostic data (step S3). After executing the process of step S3, the processor 21 terminates execution of the procedure in the flowchart in FIG. 4.

Upon acquiring the self-diagnostic data, the processor 21 uploads the self-diagnostic data to the server 30. The server controller 31 of the server 30 diagnoses the state of the analysis apparatus 10 based on the self-diagnostic data, and outputs a diagnostic result.

The processor 21 may acquire the self-diagnostic data immediately before the analysis apparatus 10 starts operations in an operation period of either the continuous observation mode or the time lapse mode. The processor 21 may acquire the self-diagnostic data immediately before the analysis apparatus 10 starts the operations, by controlling the analysis apparatus 10 to start the operations after the acquisition of the self-diagnostic data. The processor 21 may acquire the self-diagnostic data immediately after the analysis apparatus 10 terminates the operations in the operation period of either the continuous observation mode or the time lapse mode.

### <Summary>

As described above, according to the monitoring system 1, the control apparatus 20, and the server 30 of the present embodiment, self-diagnostic data is acquired within a self-diagnostic possible period during which the analysis apparatus 10 is not in a state of executing operations of observing a target object. Acquiring the self-diagnostic data within the self-diagnostic possible period reduces an impact of operations of acquiring the self-diagnostic data on an observation result of the target object. In addition, the state of the analysis apparatus 10 is diagnosed based on the self-diagnostic data, and a diagnostic result of the state of the analysis apparatus 10 is output. By outputting the diagnostic result of the state of the analysis apparatus 10, the state of the analysis apparatus 10 is more easily grasped than when the state of the analysis apparatus 10 is grasped from the self-diagnostic data alone. As a result, the state of the analysis apparatus 10 is maintained easily.

### (Other Embodiments)

Other embodiments of the monitoring system 1 will be described below.

### <Output of Log>

The analysis apparatus 10 may generate a log that records operations of the microscope 11, the camera 12, the stage 14, or the like of the analysis apparatus 10 during periods of executing observation of the target object. The analysis apparatus 10 may generate an error log that records an error when the error occurs in the operations of the microscope 11, the camera 12, the stage 14, or the like. The control apparatus 20 may acquire the log or the error log from the analysis apparatus 10, and upload the log or the error log to the server 30. The control apparatus 20 may, for example, upload the log or the error log of the analysis apparatus 10 to the server 30 every 24 hours. The intervals at which the control apparatus 20 uploads the log or the error log may be set as appropriate, not limited to 24 hours. Since the control apparatus 20 acquires the log or the error log from the analysis apparatus 10 and uploads the log or the error log to the server 30, the user of the analysis apparatus 10 or the person in charge of maintenance of the analysis apparatus 10 can check the operating state of the analysis apparatus 10, without operating the control apparatus 20 or the analysis apparatus 10.

The server controller 31 of the server 30 may analyze, by the log analyzer 314, the log or the error log of the analysis apparatus 10. The log analyzer 314 may determine whether at least one item included in the log of the analysis apparatus 10 is out of a log monitoring criterion. For example, when operations of each part of the analysis apparatus 10 are recorded as numerical values, the log monitoring criterion may be set as a numerical range when each part is operating normally. Upon acquiring the error log, the log analyzer 314 may determine that the analysis apparatus 10 is out of the log monitoring criterion.

When at least one item included in the log of the analysis apparatus 10 is out of the log monitoring criterion, the server controller 31 may notify outside the network 40 connected to the analysis apparatus 10 of an analysis result of the log. This allows the user of the analysis apparatus 10 or the person in charge of maintenance of the analysis apparatus 10 can check an abnormality contained in the log of the analysis apparatus 10, without connecting to the network 40 connected to the analysis apparatus 10. As a result, the state of the analysis apparatus 10 can be maintained easily.

### <Example of Configuration of Apparatuses>

As described above, in the monitoring system 1 according to the present disclosure, the server 30 processes data on the analysis apparatus 10. The control apparatus 20 may be configured to execute the functions of the server 30. In other words, the server 30 and the control apparatus 20 may be configured integrally.

In addition, when the control apparatus 20 is included in the analysis apparatus 10, the analysis apparatus 10 may be configured to execute the functions of the server 30. In other words, the server 30 and the analysis apparatus 10 may be configured integrally.

The control apparatus 20 and the analysis apparatus 10 may be connected in a one-to-one correspondence. In other words, the single control apparatus 20 may be connected to the single analysis apparatus 10. The server 30 may be connected to each combination of the control apparatus 20 and the analysis apparatus 10.

The single control apparatus 20 may be connected to multiple analysis apparatuses 10. In this case, the server 30 may be connected to the single control apparatus 20 via the network 40. When the network 40 is a closed network, connecting the server 30 to the single control apparatus 20 reduces the number of devices connected to the closed network.

### <Estimation of State of Analysis Apparatus 10>

As described above, in the monitoring system 1 according to the present disclosure, the server 30 can diagnose the state of the analysis apparatus 10 based on the self-diagnostic data on the analysis apparatus 10. The server 30 may estimate the future state of the analysis apparatus 10 based on the self-diagnostic data on the analysis apparatus 10. For example, the server controller 31 of the server 30 may estimate a schedule to replace the light source of the laser light, based on a measurement result of the intensity of the laser light and operating time of the light source of the laser light. The server controller 31 may also estimate a schedule for maintenance of the stage 14, based on the movement accuracy of the stage 14.

The server controller 31 may estimate a schedule for maintenance of each part of the analysis apparatus 10, based on a change in each item of the self-diagnostic data on the analysis apparatus 10 with a lapse of time.

The embodiment according to the present disclosure has been described above with reference to the drawings, but the specific configuration is not limited to this embodiment, and various modifications are included within the scope of not deviating from the gist of the present disclosure.

## Claims

1. A monitoring method for monitoring an analysis apparatus (10) configured to observe a target object, the monitoring method comprising:
acquiring self-diagnostic data on the analysis apparatus (10) in a self-diagnostic possible period during which the analysis apparatus (10) is in a state of not executing observation of the target object;
diagnosing a state of the analysis apparatus (10) based on the self-diagnostic data; and
outputting a diagnostic result of the state of the analysis apparatus (10).

2. The monitoring method according to claim 1, wherein the self-diagnostic possible period includes a period during which the analysis apparatus (10) is not set to an observation mode.

3. The monitoring method according to claim 1 or 2, wherein when the analysis apparatus (10) is operating in a continuous observation mode that is a mode of continuously executing observation of the target object, the self-diagnostic possible period includes a period before the analysis apparatus (10) starts operations in the continuous observation mode, or a period after the analysis apparatus (10) terminates the operations in the continuous observation mode.

4. The monitoring method according to claim 1 or 2, wherein when the analysis apparatus (10) is operating in a time lapse mode that is a mode of intermittently executing observation of the target object, the self-diagnostic possible period includes at least part of a period before the analysis apparatus (10) starts a series of operations to be executed in a section of a period during which the analysis apparatus (10) is operating in the time lapse mode, or at least part of a period after the analysis apparatus (10) terminates the series of operations.

5. The monitoring method according to any one of claims 1 to 4, wherein the self-diagnostic data is acquired immediately after the self-diagnostic possible period is started, or immediately before the self-diagnostic possible period is terminated.

6. The monitoring method according to any one of claims 1 to 5, further comprising:
accepting input from a user of the analysis apparatus (10) to set time of acquiring the self-diagnostic data,
wherein the self-diagnostic data is acquired at or after the time set by the user of the analysis apparatus (10), and acquired within the self-diagnostic possible period.

7. The monitoring method according to any one of claims 1 to 6, further comprising acquiring, as the self-diagnostic data, data that can be measured by setting a jig (50) on the analysis apparatus (10).

8. The monitoring method according to claim 7, wherein
the jig (50) is provided with an aperture (52), and
when the analysis apparatus (10) includes a camera (12), the aperture (52) is configured to enable analysis of noise contained in an image generated by the camera (12), or dust or dirt adhering to the camera (12), by imaging the aperture (52) by the camera (12).

9. The monitoring method according to claim 7 or 8, wherein
the jig (50) includes a pattern area (53) displaying a specific pattern, and
when the analysis apparatus (10) includes a camera (12) and a stage (14) on which the target object can be placed, the pattern area (53) is configured to enable generation of an image to check movement accuracy of the stage (14), by placing the jig (50) on the stage (14), moving the stage (14) to multiple positions, and imaging, by the camera (12), the specific pattern of the pattern area (53) when the stage (14) is moved to each of the positions.

10. The monitoring method according to any one of claims 7 to 9, wherein
the jig (50) is provided with a holder (51), and
when the analysis apparatus (10) has an autofocus function, the holder (51) is configured to enable placement of a sample used by the analysis apparatus (10) to acquire an autofocus optical signal.

11. The monitoring method according to any one of claims 1 to 10, wherein the self-diagnostic data includes data measured by a sensor (13) provided in the analysis apparatus (10).

12. The monitoring method according to claim 11, wherein the sensor (13) includes at least one of an illuminance sensor (131) configured to measure intensity of laser light used by the analysis apparatus (10) to observe the target object, a temperature sensor (132) configured to measure temperature inside a chamber in which the target object is placed, or a gas sensor (133) configured to measure gas concentration inside the chamber.

13. The monitoring method according to any one of claims 1 to 12, further comprising notifying outside a network (40) connected to the analysis apparatus (10) of a result of analyzing the state of the analysis apparatus (10) in the diagnostic result.

14. The monitoring method according to any one of claims 1 to 13, further comprising notifying outside a network (40) connected to the analysis apparatus (10) of the diagnostic result.

15. The monitoring method according to any one of claims 1 to 14, further comprising notifying a user of the analysis apparatus (10) of the diagnostic result by displaying the diagnostic result.

16. The monitoring method according to any one of claims 1 to 15, further comprising notifying a user of the analysis apparatus (10) of a result of analyzing the state of the analysis apparatus (10) in the diagnostic result.

17. The monitoring method according to any one of claims 1 to 16, further comprising:
acquiring a log that records operations of the analysis apparatus (10) in a period during which the analysis apparatus (10) is observing the target obj ect; and
outputting the log.

18. The monitoring method according to claim 17, further comprising, when at least one item included in the log is out of a log monitoring criterion, notifying outside a network (40) connected to the analysis apparatus (10) of an analysis result of the log.

19. A monitoring program configured to cause a processor to monitor an analysis apparatus (10) configured to observe a target object, the monitoring program configured to cause the processor to execute operations, the operations comprising:
acquiring self-diagnostic data on the analysis apparatus (10), outside an observation period of the target object by the analysis apparatus (10);
diagnosing a state of the analysis apparatus (10) based on the self-diagnostic data; and
outputting a diagnostic result of the state of the analysis apparatus (10).

20. A monitoring apparatus configured to monitor an analysis apparatus (10) configured to observe a target object, the monitoring apparatus configured to:
acquire self-diagnostic data on the analysis apparatus (10) in a self-diagnostic possible period during which the analysis apparatus (10) is in a state of not executing observation of the target object;
diagnose a state of the analysis apparatus (10) based on the self-diagnostic data; and
output a diagnostic result of the state of the analysis apparatus (10).
